# EUROPEAN PATENT APPLICATION

(11) **EP 4 725 419 A1**
(43) Date of publication of application: **15.04.2026**
(21) Application number: 24205836.0
(22) Date of filing: 10.10.2024
(51) Int. Cl.: A61B 5/349, A61B 5/00, A61B 5/346

(54) **MONITORING CARDIAC FUNCTION**

(71) Applicant: Koninklijke Philips N.V., 5656 AG Eindhoven (NL)
(72) Inventor: BONOMI, Alberto Giovanni, Eindhoven (NL)
(74) Representative: Philips Intellectual Property & Standards

(57) **Abstract**

Proposed concepts thus aim to provide schemes, solutions, concepts, designs, methods and systems pertaining to monitoring cardiac function of a subject. In particular, embodiments aim to provide a method for monitoring cardiac function of a subject by utilizing signal quality indicators (SQIs) to identify the best available ECG leads to use during an initialization/calibration phase of a trained algorithm for monitoring cardiac function (e.g., a machine-learning model trained to detect cardiac arrhythmia in a subject), as well as triggering a re-initialization/recalibration if the ECG lead signals are absent of transient noise (thus indicating a non-temporary change in the monitoring setup).

## Description

### FIELD OF THE INVENTION

This invention relates to the field of monitoring cardiac function of a subject.

### BACKGROUND OF THE INVENTION

Electrocardiograms (ECGs) are routinely used to monitor cardiac electrical activity. During an ECG, electrode sensors are placed on the skin to measure small scale electrical signals that originate from the heart. ECG measurements are incorporated in numerous care pathways and are beneficial for the diagnosis of cardiac diseases and the identification of cardiac anomalies. In clinical practice, multiple ECG signals may be obtained during a single ECG examination. Typical ECG monitors involve multiple ECG channels (or leads) which are each associated with a different ECG sensor placed on the patient's skin and which each have their own associated ECG signal.

There are many ways in which ECG signals may be used for patient monitoring, and these different methods may have different ECG channel configuration requirements. Cardiac diagnosis typically requires 12-lead ECG recordings, or 1-3 ECG leads Holter monitoring. In critical care and telemetry wards patients are typically monitored using 4 ECG lead systems for real-time arrythmia detection and patient surveillance.

Noise is often present in ECG signals as the ECG comprises small scale electrical impulses that may be easily affected by patient movement or by motion of the ECG wires. Different leads of an ECG monitor may suffer different levels of noise and therefore the different ECG signals obtained during an ECG exam may vary in their quality. In addition, physiological characteristics of the patient and the positioning of the electrodes on the body may generate ECG waveforms that are difficult to interpret, despite the absence of noise.

ECG waveforms are typically interpreted by clinical experts who visually inspect the waveform. Artificial intelligence and other interpretation algorithms may also be used to streamline expert review of ECG signals and provide automatic identification of cardiac events for alarming purposes. The accuracy of such interpretation algorithms strongly depends on the quality of the ECG waveform.

### SUMMARY OF THE INVENTION

The invention is defined by the claims.

According to examples in accordance with an aspect of the invention, there is provided a method for monitoring cardiac function of a subject.

The method comprises: determining a first signal quality indicator value for each of a plurality of ECG lead signals, each ECG lead signal comprising ECG data of the same subject; determining a first subset of the plurality of ECG lead signals based on the first signal quality indicator value for each ECG lead signal; calibrating a monitoring algorithm based on the first subset, the monitoring algorithm for monitoring cardiac function of the subject; at a later time, determining if transient noise is present in each of the ECG lead signals of the first subset; and if transient noise is absent in at least one of the ECG lead signals of the first subset: determining a second signal quality indicator value for each of the plurality of ECG lead signals; and if the second signal quality indicator value for a given ECG lead signal deviates from the first signal quality indicator value for the same ECG lead signal by more than a predetermined threshold: determining a second subset of the plurality of ECG lead signals based on the second signal quality indicator value for each ECG lead signal; and recalibrating the monitoring algorithm based on the second subset.

Proposed concepts thus aim to provide schemes, solutions, concepts, designs, methods and systems pertaining to monitoring cardiac function of a subject. In particular, embodiments aim to provide a method for monitoring cardiac function of a subject by utilizing signal quality indicators (SQIs) to identify the best available ECG leads to use during an initialization/calibration phase of a trained algorithm for monitoring cardiac function (e.g., a machine-learning model trained to detect cardiac arrhythmia in a subject), as well as triggering a re-initialization/recalibration if the ECG lead signals are absent of transient noise (thus indicating a non-temporary change in the monitoring setup).

In other words, it is proposed that by using SQIs, the best available ECG leads to use for a calibration/initialization phase of a trained algorithm can be identified and then by monitoring the presence of transient noise in said ECG leads, an effective method of knowing when to trigger a recalibration is provided. The above thus allows an interpretation algorithm (the trained algorithm) to process the ECG waveforms with the most favourable characteristics (physiological and/or noise characteristics) to find a stable normal pattern, such that abnormalities can be more reliably detected.

Additionally, by monitoring transient noise in the ECG leads, an efficient and reliable way of detecting a systematic (long standing) change in the monitoring setup (i.e., the ECG leads) is provided, and thus this provides a suitable trigger for recalibrating the trained algorithm. For example, if the ECG leads have been moved or repositioned on a subject, then the subset of optimal ECG leads identified in the first place may now be sub-optimal, and so a new subset should thus be identified and used to recalibrate/re-initialize the trained algorithm to ensure continued effective monitoring.

This invention may be of particular use, for example, in monitoring cardiac arrythmia. Ultimately, an improved method for monitoring cardiac function of a subject is provided.

In some embodiments, the method may further comprise: storing the first signal quality indicator value for each of the first subset as a baseline value for each of the first subset; measuring, at a later time to the determining of the first signal quality indicator values, a subsequent signal quality indicator value for each of the first subset; comparing the baseline value and the subsequent signal quality indicator value for each of the first subset; determining a further subset of the first subset of the plurality of ECG lead signals based on the comparison; determining if transient noise is present in each of the further subset; and if transient noise is present in at least one of the further subset, updating the baseline values to comprise the subsequent signal quality indicator values for each of the first subset. This allows for baseline values to be stored for each of the subset of ECG lead signals being used (thus allowing changes in the signals to be efficiently determined) while also allowing for an updating of said baseline values if the change is not due to a systematic change, i.e., is a temporary change, (indicated by the presence of transient noise).

In some embodiments, determining the further subset based on the comparison may comprise selecting ECG lead signals with a deviation between their baseline value and their subsequent signal quality indicator value above a predetermined threshold. This provides an effective and efficient method of determining ECG leads signals which have had substantial changes in their values.

In some embodiments, the predetermined threshold may be user-adjustable. This may allow a user to influence when the baseline values might need updating.

In some embodiments, the monitoring algorithm may be for detecting cardiac arrhythmia in the subject. This may be a particularly beneficial use of the invention and one for which it is particularly effective.

In some embodiments, a signal quality indicator may comprise at least one physiological indicator and/or at least one noise indicator. These may be particularly effective indicators for indicating the signal quality of an ECG lead signal for cardiac monitoring.

In some embodiments, a physiological indicator may comprise at least one of: a QRS amplitude; an amplitude difference between a QRS complex and a surrounding waveform of the ECG signal; a QRS polarity; a slope of a QRS complex; a time-variation of QRS polarity; and a difference between Q, R, and/or S wave peaks and T and/or P wave peaks. These may all be particularly effective physiological indicators for indicating a signal quality of an ECG lead signal.

In some embodiments, a noise indicator may comprise at least one of: transient noise artefacts; electrode noise; presence of noise in predetermined frequency bands; electromyographic interference; baseline wander; and power line noise. These may all be particularly effective noise indicators for indicating a signal quality of an ECG lead signal.

In some embodiments, the first signal quality indicator for each of the plurality of ECG lead signals may comprise at least one physiological indicator and at least one noise indicator. This may allow multiple distinct aspects of the ECG lead signal's quality to be considered.

In some embodiments, the method may further comprise using the monitoring algorithm to monitor cardiac function of the subject using the first subset. This may allow the selected subset of ECG lead signals to actually be used to monitor the cardiac function of a subject.

According to another aspect of the invention, there is provided a computer program comprising code means for implementing the method of any herein disclosed method when said program is run on a processing system.

According to another aspect of the invention, there is provided a system for monitoring cardiac function of a subject. The system comprising a processing arrangement configured to: determine a first signal quality indicator value for each of a plurality of ECG lead signals, each ECG lead signal comprising ECG data of the same subject; determine a first subset of the plurality of ECG lead signals based on the first signal quality indicator value for each ECG lead signal; and calibrate a monitoring algorithm based on the first subset, the monitoring algorithm for monitoring cardiac function of the subject; at a later time, determine if transient noise is present in each of the ECG lead signals of the first subset; and if transient noise is absent in at least one of the ECG lead signals of the first subset: determine a second signal quality indicator value for each of the plurality of ECG lead signals; and if the second signal quality indicator value for a given ECG lead signal deviates from the first signal quality indicator value for the same ECG lead signal by more than a predetermined threshold: determine a second subset of the plurality of ECG lead signals based on the second signal quality indicator value for each ECG lead signal; and recalibrate the monitoring algorithm based on the second subset.

In some embodiments, the system may further comprise a plurality of ECG leads applied to the subject, each configured to provide one of the plurality of ECG lead signals.

In some embodiments, the plurality of ECG leads may be applied to the subject according to a standardized ECG electrode arrangement.

Thus, there may be proposed concepts for monitoring cardiac function of a subject, and this may be done based on determining signal quality indicator values for ECG lead signals and then calibrating a monitoring algorithm based on a subset of the ECG leads signals, chosen based on their signal quality indicator values. Furthermore, a recalibration of the monitoring algorithm can be triggered at a later time if transient noise is found to be absent in at least one of the subset of ECG lead signals.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flow diagram of a method for monitoring cardiac function of a subject according to a proposed embodiment;
Fig. 2 is an illustration depicting a visual representation of an assessment of signal quality of ECG lead signals and a period of transient noise;
Fig. 3 is a flow diagram of a method for monitoring cardiac function of a subject according to a proposed embodiment;
Fig. 4 is a simplified block diagram of a system for monitoring cardiac function of a subject according to a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

Implementations in accordance with the present disclosure relate to various techniques, methods, schemes and/or solutions pertaining to monitoring cardiac function of a subject. According to proposed concepts, a number of possible solutions may be implemented separately or jointly. That is, although these possible solutions may be described below separately, two or more of these possible solutions may be implemented in one combination or another.

Embodiments of the invention aim to provide a method for monitoring cardiac function of a subject. This can be achieved by utilizing signal quality indicators (SQIs) to identify the best available ECG leads to use during an initialization/calibration phase of a trained algorithm for monitoring cardiac function (e.g., a machine-learning model trained to detect cardiac arrhythmia in a subject), as well as triggering a re-initialization/recalibration if the ECG lead signals are absent of transient noise (thus indicating a non-temporary change in the monitoring setup).

In other words, it is proposed that by using SQIs, the best available ECG leads to use for a calibration/initialization phase of a trained algorithm can be identified and then by monitoring the presence of transient noise in said ECG leads, an effective method of knowing when to trigger a recalibration is provided. The above thus allows an interpretation algorithm (the trained algorithm) to process the ECG waveforms with the most favourable characteristics (physiological and/or noise characteristics) to find a stable normal pattern, such that abnormalities can be more reliably detected.

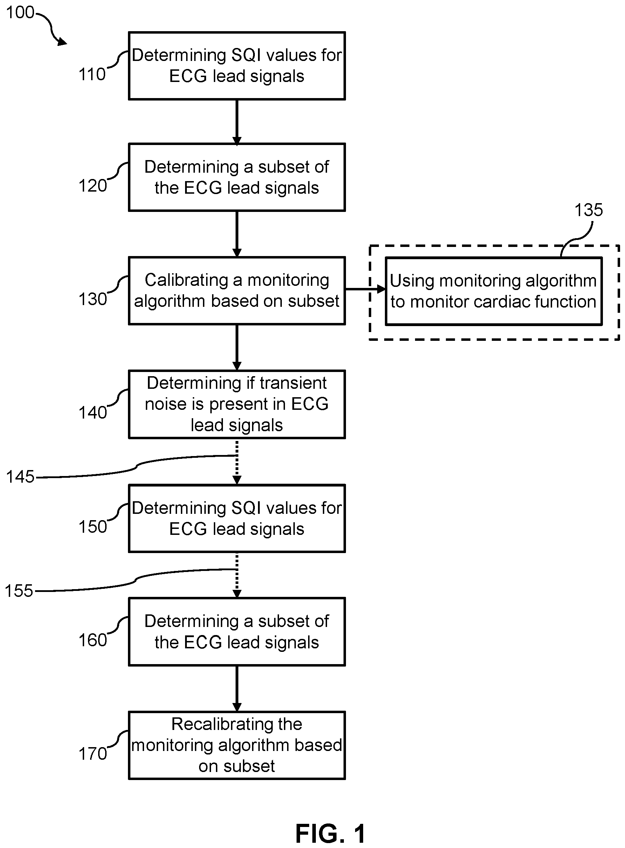

Referring now to Fig. 1, there is depicted a flow diagram of a method 100 for monitoring cardiac function of a subject according to a proposed embodiment.

The method 100 begins with step 110 comprising determining a first signal quality indicator value for each of a plurality of ECG lead signals, each ECG lead signal comprising ECG data of the same subject. In other words, each of the plurality of ECG lead signals are from (i.e., generated by) ECG leads applied to the same subject. A signal quality indicator value of the present invention can be any qualitative or quantitative value indicating the signal quality of the ECG lead signal and its suitability for use in cardiac monitoring. For example, the signal quality indicator value may be a positive number, wherein the magnitude of the determined signal quality value is greater for better quality signals. In another example, the signal quality indicator value may be a qualitative description of the ECG signal quality, for example the signal quality indicator value may comprise a categorisation of the ECG signal into one of poor quality, medium quality, or high quality. In other words, step 110 comprises determining a first value of a first signal quality indicator for each of a plurality of ECG lead signals, each ECG lead signal comprising ECG data of the same subject.

In this embodiment, a signal quality indicator comprises at least one physiological indicator and/or at least one noise indicator. These are particularly effective indicators for indicating the signal quality of an ECG lead signal for cardiac monitoring. In other words, in this embodiment, the first signal quality indicator value for each of the ECG lead signals comprise at least one physiological indicator and/or at least one noise indicator. Of course, in other embodiments, a signal quality indictor can comprise any suitable indicator for indicating signal quality, as would be understood by the skilled person.

In fact, in this embodiment, the first signal quality indicator for each of the plurality of ECG lead signals comprise at least one physiological indicator and at least one noise indicator. This allows multiple distinct aspects of the ECG lead signal's quality to be considered. To be clear, in this embodiment, the first signal quality indicator value for each of the ECG lead signals would thus comprise a first physiological indicator value and a first noise indicator value.

For example, in this embodiment, a physiological indicator comprises at least one of: a QRS amplitude; an amplitude difference between a QRS complex and a surrounding waveform of the ECG signal; a QRS polarity; a slope of a QRS complex; a time-variation of QRS polarity; and a difference between Q, R, and/or S wave peaks and T and/or P wave peaks. These are all particularly effective physiological indicators for indicating a signal quality of an ECG lead signal, however, as would be appreciated by the skilled person, in other embodiments, a physiological indicator can comprise any suitable parameter related to a physiological aspect of the subject.

A QRS amplitude can be understood as an amplitude of a QRS complex. A QRS polarity can be understood as a QRS complex polarity. A difference between Q, R and/or S wave peaks and T and/or P wave peaks can be understood as a difference between at least one of: a Q wave peak and a T wave peak; a Q wave peak and a P wave peak; a R wave peak and a T wave peak; a R wave peak and a P wave peak; an S wave peak and a T wave peak; an S wave peak and a P wave peak.

For example, in this embodiment, a noise indicator comprises at least one of: transient noise artefacts; electrode noise; presence of noise in predetermined frequency bands; electromyographic interference; baseline wander; and power line noise. These are all particularly effective noise indicators for indicating a signal quality of an ECG lead signal, however, as the skilled person would appreciate, in other embodiments, a noise indicator can comprise any suitable parameter related to noise within an ECG lead signal.

Transient noise artifacts can be understood as sudden, short-duration disturbances in the ECG signal often caused by patient movement or temporary electrical interference. Electrode noise can be understood as fluctuations in the ECG signal resulting from poor electrode-skin contact or deterioration of the electrode material. Presence of noise in predetermined frequency bands can be understood as interference occurring within specific frequency ranges, often indicating particular types of environmental or physiological disturbances. Electromyographic interference can be understood as high-frequency noise in the ECG signal caused by electrical activity from skeletal muscle contractions. Baseline wander can be understood as low-frequency deviation of the ECG signal's baseline, typically due to patient respiration or movement. Power line noise can be understood as regular, high-frequency interference in the ECG signal caused by electromagnetic fields from nearby electrical power sources, usually at 50 or 60 Hz.

It should be mentioned that the plurality of ECG lead signals may be acquired in the first place via any suitable monitoring system (as would be apparent to the skilled person) such as a Holter monitoring system, a telemetry monitoring system, or a bedside monitoring system.

Step 120 comprises determining a first subset of the plurality of ECG lead signals based on the first signal quality indicator value for each ECG lead signal. For example, only the higher quality ECG lead signals are selected to be in the first subset. For example, for each type of signal quality indicator value there may be a corresponding predetermined threshold above which (or below which, depending on the specific type of signal quality indicator value, as would be understood by the skilled person) the ECG lead signal is put in the first subset. If multiple signal quality indicator values are being determined for each ECG lead signal, in some embodiments, all of the indicator values for each ECG lead signal may have to indicate that the ECG lead signal is high quality for the ECG lead signal to be put in the first subset, whereas in other embodiments, only one of the indicator values indicating that the ECG lead signal is high quality may be sufficient for the ECG lead signal to be put in the first subset. In some embodiments, the predetermined threshold for each type of signal quality indicator value may vary dependent on the number of available ECG lead signals and/or the number of ECG lead signals being selected for the first subset (e.g., during a first round of selection). For example, if in a first round of selection, only one of the ECG lead signals is sufficiently high quality to be selected to be put in the first subset, there may be a second round of selection with lower thresholds/requirements such that more ECG lead signals can be selected to be put in the first subset. As the skilled person would appreciate, there are many ways in which the first subset could be selected using the first SQI values of the ECG lead signals, however, the important aspect is that it is the higher quality ECG lead signals which are selected.

More complicated selection algorithms (for the first subset) are also envisioned which utilise the determined signal quality indicator values alongside other known quantities such as physiological parameters of the patient, parameters of the ECG monitoring set up, and details of the further processing steps and diagnosis procedures that are to be carried out using the selected ECG signal(s).

In a simple example, the first subset may include ECG lead signals (from the plurality of ECG lead signals) which are characterized by the least amount of baseline noise and show tall and sharp QRS complexes - these are particularly useful SQIs when selecting the best ECG lead signals for arrhythmia detection.

In step 130, a monitoring algorithm is calibrated based on the first subset, the monitoring algorithm for monitoring cardiac function of the subject. In some embodiments, the monitoring algorithm can be specifically configured for monitoring cardiac function of a subject, e.g., it can comprise a machine-learning algorithm specifically trained to monitor cardiac function of a subject.

In this embodiment, however, the monitoring algorithm is, in fact, specifically for detecting cardiac arrhythmia in the subject (e.g., it may be a trained machine-learning model specifically trained to detect cardiac arrhythmia from ECG lead signals). This is a particularly beneficial use of the invention and one for which it is particularly effective, however, in other embodiments, the monitoring algorithm can alternatively or additionally be for monitoring any other suitable cardiac function such as sinus rhythm, tachycardia, bradycardia, atrial fibrillation, ventricular fibrillation, etc.

Calibration of the monitoring algorithm can also be understood as performing a calibration/learning/initialization phase of the monitoring algorithm.

In some embodiments, the method 100 further includes step 135 of using the monitoring algorithm to monitor cardiac function of the subject using the first subset of ECG lead signals. This thus allows the selected subset of ECG lead signals to actually be used to monitor the cardiac function of a subject.

The method 100 also includes step 140 of, at a later time (i.e., to step 130), determining if transient noise is present in each of the ECG lead signals of the first subset. Transient noise in an ECG lead signal refers to sudden, short-duration (e.g., under three seconds) disturbances or artifacts that appear in the ECG waveform. Transient noise is non-rhythmic, non-repeating, and discontinuous noise. As the skilled person would understand, the presence of transient noise can be determined by measuring characteristics of an ECG lead signal which are related to high frequency noise and the consistency/stability of noise indicators over time. It should be noted that the noise indicators described above can also be used to wholly or partially determine if transient noise is present in each of the ECG lead signals.

If transient noise is absent in at least one of the ECG lead signals of the first subset, indicated by dashed arrow 145, the method moves on to step 150. Step 150 is substantially the same as step 110, however, performed at a later time.

Step 150 comprises determining a second signal quality indicator value for each of the plurality of ECG lead signals. For example, the second signal quality indicators can be the same as the first signal quality indicators (e.g., comprising the same physiological and/or noise indicators).

If the second signal quality indicator value for a given ECG lead signal (i.e., at least one ECG lead signal) deviates from the first signal quality indicator value for the same ECG lead signal by more than a predetermined threshold, indicated by dashed arrow 155, then the method moves on to steps 160 and 170. Steps 160 and 170 are substantially the same as steps 120 and 130 respectively, however, performed at a later time.

For example, the predetermined threshold may comprise (the mean value of) the first signal quality indicator value for the given ECG lead signal plus or minus a standard deviation (e.g., observed during the determining of the first signal quality indicator value for the given ECG lead signal). In other embodiments, the predetermined threshold may be, for example, a plus or minus 10% change, or any other suitable predetermined threshold for essentially indicating that there has been a substantial change in the SQI value for the ECG lead signal. It would be perfectly clear to the skilled person that the predetermined threshold could take many different forms depending on the situation and the specific SQI value. For instance, in some embodiments, each type of SQI value may have its own predetermined threshold.

Step 160 comprises determining a second subset of the plurality of ECG lead signals based on the second signal quality indicator value for each ECG lead signal. In some situations, the second subset can be the same as the first subset but in other situations it can be different.

Step 170 comprises recalibrating the monitoring algorithm based on the second subset. For example, recalibration can be substantially the same as calibration, however, performed at a later time. Recalibrating can thus also be understood as performing a recalibration/relearning phase of the monitoring algorithm (e.g., a trained machine learning model).

Monitoring and diagnostic ECG systems use multiple ECG leads for real-time interpretation of cardiac characteristics and, for example, arrhythmias. Selecting ECG leads which are of high quality for human and machine interpretation is challenging as noise and signal artefacts can worsen algorithm performance accuracy. Algorithms are often designed to capture the normal ECG pattern in a subject during a calibration/initialization/leaming phase/period to facilitate the discovery of meaningful deviation from the normal pattern, thereby facilitating, for example, the detection of arrhythmias.

As described, the present disclosure therefore proposes to utilize signal quality indicators (SQIs) to identify the best available ECG leads during a calibration/initialization/learning phase/period of a monitoring algorithm as well as to trigger a re-initialization (re-calibration / re-learning) after the SQI features display a substantial change over time in the absence of transient noise. This would therefore facilitate the monitoring algorithm processing the ECG waveforms with the most favourable characteristics to find a stable normal pattern.

This concept can be supported by the fact that SQI features describing physiological ECG characteristics are relatively stable over time and are mainly influenced by lead type in the absence of transient noise. For example, an SQI (physiological) feature describing the amplitude difference between R peaks and non-R peaks typically shows a larger variability across leads than over time. This therefore suggests that during a learning phase (i.e., a calibration period), the monitoring algorithm could identify which are the optimal ECG leads for processing/monitoring. In other words, the SQI variation over time is often (much) smaller than the between-lead difference in the mean feature value - thus, SQI feature values tend to remain stable over time for a certain lead (in the absence of transient noise).

When SQI features deviate from the normal distribution, the proposed method can also trigger a re-calibration of the monitoring algorithm. This can thus help identify when changes in skin electrode contacts, posture (supine, prone, sitting, etc.), or respiratory behaviour have occurred and may impact the normal characteristics of the ECG signal. In fact, factors causing relative movements between the chest surface and the heart (or the ventricular axis) can change the ECG signal morphology thus also requiring the algorithm to be re-calibrated in order to continue optimal performance.

In an example of the disclosure, the main elements of the inventive method can be understood as: acquiring and monitoring multiple ECG lead signals of a subject over time; measuring signal quality of the ECG lead signals related to physiological characteristics such as QRS amplitude, QRS polarity, etc.; measuring signal quality of the ECG lead signals related to transient noise artefacts such as electrode noise, electromyographic interferences, baseline wander, power line noise, etc.; utilizing the physiological SQIs to select the best available lead for real-time cardiac monitoring (such as arrhythmia detection) during a calibration/learning/initialization phase of the monitoring algorithm; utilizing noise and physiological SQIs to identify whether the monitoring algorithm should be recalibrated/re-learned/re-initialized due to changes in patient conditions (and not merely transient noise); and using the ECG leads selected by the calibration phase of the monitoring algorithm for post-processing interpretation and visualization.

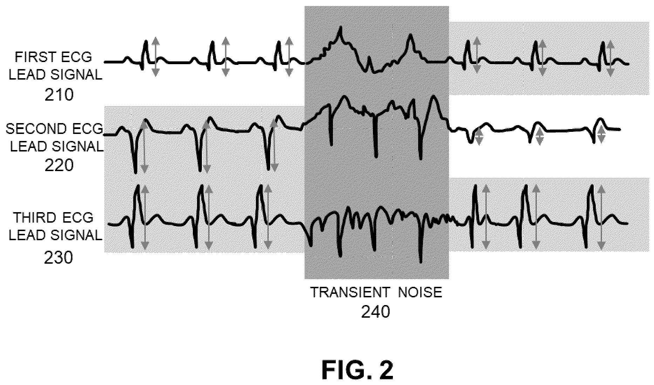

Referring now to Fig. 2, there is depicted an illustration of a visual representation of an assessment of signal quality of ECG lead signals and a period of transient noise.

Fig. 2 shows a first ECG lead signal 210, a second ECG lead signal 220, and a third ECG lead signal 230 describing a subject's cardiac activity over a given time period. For example, the signal quality of the first, second, and third ECG signals can be assessed by comparing the values of QRS amplitude detected by each of the leads (indicated by the vertical double-ended arrows).

ECG signal quality for the first, second, and third ECG lead signals can be assessed based on the calculated QRS amplitude values for each. Larger QRS amplitude is typically indicative of a better-quality signal. When the ECG signal quality is assessed using the QRS amplitude of all the beats that have been detected in the three ECG signals, the second and third ECG lead signals, 220 and 230, are identified as having better signal quality compared to the first ECG lead signal 210, since the average values of the QRS amplitudes for the second and third ECG lead signals are greater for the first ECG lead signal. The second and third ECG lead signals 220 and 230 can thus be used for a calibration phase of a monitoring algorithm.

During an identified period of transient noise 240 (for example, due to patient movement or rotation), the QRS amplitudes for each ECG lead are not considered (i.e., ignored) for the purpose of determining whether to initiate a recalibration of the monitoring algorithm (i.e., determining whether there has been a substantial change in the ECG leads' signal quality indicator values). Following the period of transient noise 240, the QRS amplitudes for the second ECG lead 220 can be seen to have changed substantially (e.g., deviating by more than a standard deviation of the period before the transient noise and/or deviating by more than 10%) when compared to before the transient noise. In this case, due to the substantial deviation in the SQI value of at least one of the ECG lead signals (now in the absence of transient noise), the monitoring algorithm can be re-calibrated (re-initialized / re-learned) on a new subset of optimal leads (e.g., now the first and third ECG lead signals, 210 and 230, due to their larger QRS amplitudes compared to the second ECG lead signal 220).

However, when considering only normal heartbeats, it is clear that the second ECG lead signal 220 is of better quality than the first ECG signal 210. Typically, the quality of the portion of an ECG signal corresponding to normal heartbeats is more indicative of the usefulness of the signal for monitoring and diagnosis than the portions of the signal corresponding to other cardiac events. Therefore, basing the assessment of signal quality on all the identified heartbeats (including both normal and premature heart beats) may lead to an unreliable assessment of the relative quality of the two signals.

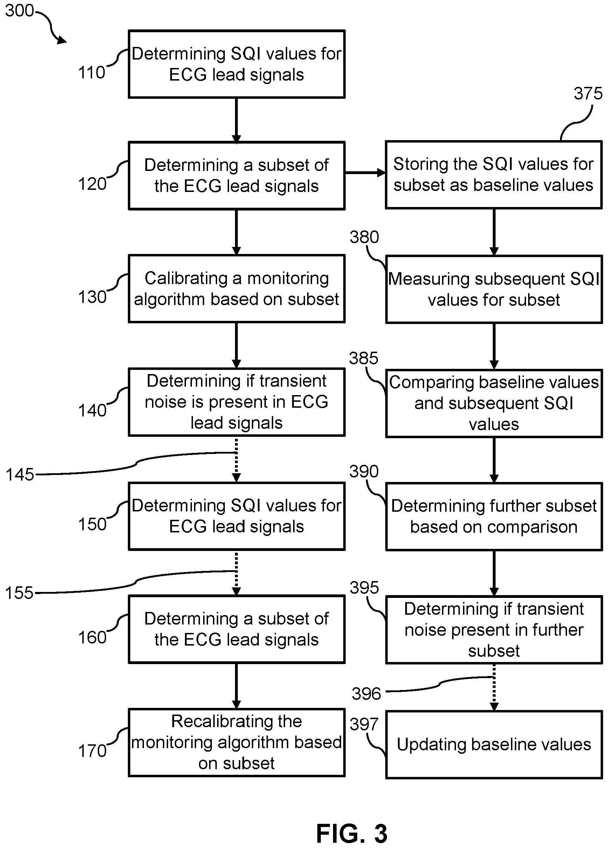

Referring now to Fig. 3, there is depicted a flow diagram of a method 300 for monitoring cardiac function of a subject according to a proposed embodiment.

Steps 110, 120, 130, 140, 145, 150, 160 and 170 are substantially the same as have been described in relation to the method 100 of Fig. 1.

Step 375 comprises storing the first signal quality indicator value for each of the first subset as a baseline value for each of the first subset. For instance, the baseline values can be stored in a memory, in the cloud, or in any other suitable form of storage, as would be appreciated by the skilled person.

Step 380 comprises measuring, at a later time to the determining of the first signal quality indicator values, a subsequent signal quality indicator value(s) (of at least the same signal quality indicator).

Step 385 comprises comparing the baseline value and the subsequent signal quality indicator value for each of the first subset (and, of course, if multiple signal quality indicators have been used, for each matching pair of signal quality indicators). Step 390 comprises determining a further subset of the first subset of the plurality of ECG lead signals based on the comparison. For example, the ECG lead signals which show the most deviation between their baseline value and their subsequent signal quality indicator value in the comparison can be selected to be put in the further subset.

In this embodiment, in fact, determining the further subset comprises selecting ECG lead signals with a deviation between their baseline value and their subsequent signal quality indicator value above a predetermined threshold. This threshold may be an absolute value or a percentage value (e.g., a deviation of more than 10%), or the ends of a range of value distribution as observed during the initial/previous calibration phase/procedure. This thus provides an effective and efficient method of determining ECG lead signals which have had substantial changes in their signal quality indicator values. In some embodiments, the predetermined threshold can be user adjustable. This could thus allow a user to influence when the baseline values might need updating (e.g., a first user could set the threshold at 10% or more and a second user could set the threshold at 20% or more, such that the second user is more tolerant of value changes in the signal quality indicators).

In essence, steps 375 to 385 can be understood as the monitoring over time of the SQI values of each ECG lead signal such that substantial changes can be identified and thus, if needed, the baseline (i.e., normal) values can be updated.

Step 395 comprises determining if transient noise is present in each of the further subset. Step 395 is thus similar to step 140, however, restricted to only those ECG lead signals in the further subset of the first subset.

If transient noise is present in at least one of the further subset, indicated by dashed arrow 396 (indicating that the changes are not long-standing systematic changes but merely due to random fluctuations), then the method moves on to step 397 of updating the baseline values to comprise the subsequent signal quality indicator values for each of the first subset. In some embodiments, the test of dashed arrow 396 can instead be: if transient noise is present in each ECG lead signal of the further subset (i.e., all of the ECG lead signals). In some embodiments, if this isn't case, i.e., if transient noise is absent in at least one of the ECG lead signals, then the method 100 can perform steps 150, 160, 170 instead of step 397 (i.e., a recalibration can be performed instead of updating the baseline values).

The above-described steps thus allow for baseline values to be stored for each of the subset of ECG lead signals being used (thus allowing changes in the signals to be efficiently determined) while also allowing for an updating of said baseline values if the change is not due to a systematic change, i.e., is merely a temporary change (indicated by the presence of transient noise).

Steps 375 to 397 can thus be understood as a parallel process to the actual monitoring of a subject using the selected leads in the first subset (such as in step 135 of method 100). It allows the system to essentially test whether the baseline/normal values of the selected leads need updating and whether the selected leads in the first subset remain the best for the monitoring algorithm to use to monitor the subject. For example, this is accomplished by measuring the SQI values for each ECG lead signal and computing the correspondence (or difference) between the current SQI values and the ones obtained during the calibration phase. In case of a large deviation being observed between the stored SQI values (e.g., the ones used during calibration) and the current SQI values for a particular ECG lead signal, the system can then measure whether transient noise is present in said particular ECG lead signal. If transient noise is detected, then the deviation of SQI values can be ignored because it is likely only temporary (and the baseline values can merely be updated). However, if transient noise is absent in the ECG lead signal, then the change can likely be attributed to a systematic (long standing) change in the monitoring setup of the ECG leads - in this case, it would thus be beneficial to re-calibrate the monitoring system (e.g., potentially using a different subset of ECG lead signals).

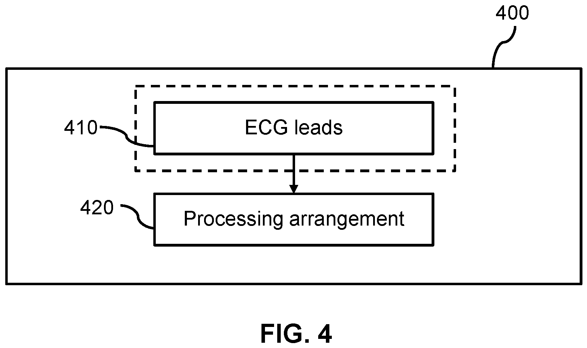

Referring now to Fig. 4, there is depicted a simplified block diagram of a system 400 for monitoring cardiac function of a subject according to a proposed embodiment.

The system 400 comprises a processing arrangement 420. The processing arrangement 420 is configured to perform any herein-disclosed method, e.g., method 100 or 300. For example, the processing arrangement 420 is configured to: determine a first signal quality indicator value for each of a plurality of ECG lead signals, each ECG lead signal comprising ECG data of the same subject; determine a first subset of the plurality of ECG lead signals based on the first signal quality indicator value for each ECG lead signal; and calibrate a monitoring algorithm based on the first subset, the monitoring algorithm for monitoring cardiac function of the subject; at a later time, determine if transient noise is present in each of the ECG lead signals of the first subset; and if transient noise is absent in at least one of the ECG lead signals of the first subset: determine a second signal quality indicator value for each of the plurality of ECG lead signals; and if the second signal quality indicator value for a given ECG lead signal deviates from the first signal quality indicator value for the same ECG lead signal by more than a predetermined threshold: determine a second subset of the plurality of ECG lead signals based on the second signal quality indicator value for each ECG lead signal; and recalibrate the monitoring algorithm based on the second subset.

In some embodiments, the system 400 further comprises a plurality of ECG leads 410 applied to the (same) subject, each configured to provide one of the plurality of ECG lead signals. For example, the plurality of ECG leads can be applied to the subject according to a standardized ECG electrode arrangement, such as a standard 12-lead ECG arrangement which include three limb leads, three augmented leads, and six precordial leads places across the chest. In another example, the standardized ECG electrode arrangement could be a simpler standardized 3-lead ECG arrangement with leads placed on the right arm, left arm, and left leg.

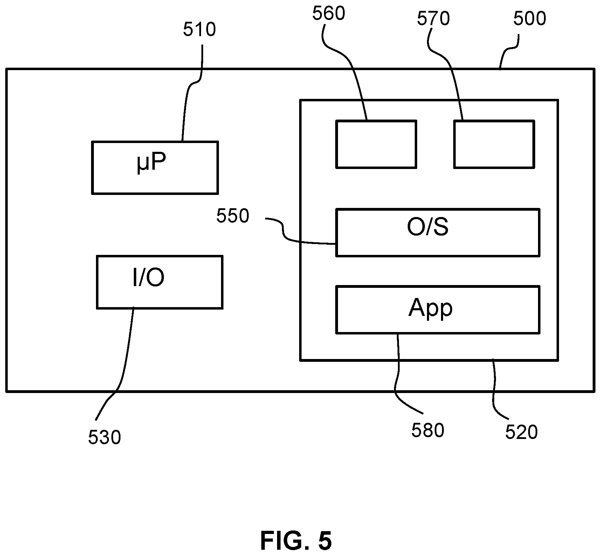

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, Python, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 500 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1 and 3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 5 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions, the architecture, functionality, and operation of possible implementations of systems, methods, and computer program products according to various embodiments of the present invention.

## Claims

1. A computer-implemented method (100) for monitoring cardiac function of a subject, the method comprising:
determining a first signal quality indicator value (110) for each of a plurality of ECG lead signals, each ECG lead signal comprising ECG data of the same subject;
determining a first subset (120) of the plurality of ECG lead signals based on the first signal quality indicator value for each ECG lead signal;
calibrating a monitoring algorithm (130) based on the first subset, the monitoring algorithm for monitoring cardiac function of the subject;
at a later time, determining if transient noise is present (140) in each of the ECG lead signals of the first subset; and
if transient noise is absent (145) in at least one of the ECG lead signals of the first subset:
determining a second signal quality indicator value (150) for each of the plurality of ECG lead signals; and
if the second signal quality indicator value for a given ECG lead signal deviates from the first signal quality indicator value for the same ECG lead signal by more than a predetermined threshold (155):
determining a second subset (160) of the plurality of ECG lead signals based on the second signal quality indicator value for each ECG lead signal; and
recalibrating the monitoring algorithm (170) based on the second subset.

2. The computer-implemented method of claim 1, wherein the method further comprises:
storing the first signal quality indicator value (375) for each of the first subset as a baseline value for each of the first subset;
measuring, at a later time to the determining of the first signal quality indicator values, a subsequent signal quality indicator value (380) for each of the first subset;
comparing the baseline value and the subsequent signal quality indicator value (385) for each of the first subset;
determining a further subset (390) of the first subset of the plurality of ECG lead signals based on the comparison;
determining if transient noise is present (395) in each of the further subset; and
if transient noise is present (396) in at least one of the further subset, updating the baseline values (397) to comprise the subsequent signal quality indicator values for each of the first subset.

3. The computer-implemented method of claim 2, wherein determining the further subset based on the comparison comprises selecting ECG lead signals with a deviation between their baseline value and their subsequent signal quality indicator value above a predetermined threshold.

4. The computer-implemented method of claim 3, wherein the predetermined threshold is user-adjustable.

5. The computer-implemented method of any of claims 1 to 4, wherein the monitoring algorithm is for detecting cardiac arrhythmia in the subject.

6. The computer-implemented method of any of claims 1 to 5, wherein a signal quality indicator comprises at least one physiological indicator and/or at least one noise indicator.

7. The computer-implemented method of claim 6, wherein a physiological indicator comprises at least one of: a QRS amplitude; an amplitude difference between a QRS complex and a surrounding waveform of the ECG signal; a QRS polarity; a slope of a QRS complex; a time-variation of QRS polarity; and a difference between Q, R, and/or S wave peaks and T and/or P wave peaks.

8. The computer-implemented method of claim 6 or 7, wherein a noise indicator comprises at least one of: transient noise artefacts; electrode noise; presence of noise in predetermined frequency bands; electromyographic interference; baseline wander; and power line noise.

9. The computer-implemented method of any of claims 6 to 8, wherein the first signal quality indicator for each of the plurality of ECG lead signals comprises at least one physiological indicator and at least one noise indicator.

10. The computer-implemented method of any of claims 1 to 9, wherein the method further comprises using the monitoring algorithm (135) to monitor cardiac function of the subject using the first subset.

11. A computer program product comprising computer program code means which, when executed on a computing device having a processing system, cause the processing system to perform all of the steps of the computer-implemented method according to any of claims 1 to 10.

12. A system (400) for monitoring cardiac function of a subject, the system comprising:
a processing arrangement (420) configured to:
determine a first signal quality indicator value for each of a plurality of ECG lead signals, each ECG lead signal comprising ECG data of the same subject;
determine a first subset of the plurality of ECG lead signals based on the first signal quality indicator value for each ECG lead signal;
calibrate a monitoring algorithm based on the first subset, the monitoring algorithm for monitoring cardiac function of the subject; and
at a later time, determine if transient noise is present in each of the ECG lead signals of the first subset; and
if transient noise is absent in at least one of the ECG lead signals of the first subset:
determine a second signal quality indicator value for each of the plurality of ECG lead signals; and
if the second signal quality indicator value for a given ECG lead signal deviates from the first signal quality indicator value for the same ECG lead signal by more than a predetermined threshold:
determine a second subset of the plurality of ECG lead signals based on the second signal quality indicator value for each ECG lead signal; and
recalibrate the monitoring algorithm based on the second subset.

13. The system of claim 12, further comprising:
a plurality of ECG leads (410) applied to the subject, each configured to provide one of the plurality of ECG lead signals.

14. The system of claim 13, wherein the plurality of ECG leads are applied to the subject according to a standardized ECG electrode arrangement.

15. The system of any of claims 12 to 14, wherein the monitoring algorithm is for detecting cardiac arrhythmia in the subject.
